**Europäisches Patentamt**

**European Patent Office**

**Office européen des brevets**

(19)

(11) Publication number: **0 093 175**

**B1**

(12) **EUROPEAN PATENT SPECIFICATION**

(45) Date of publication of patent specification: **04.01.89**

(51) Int. Cl.⁴: **A 61 F 5/44**

(21) Application number: **82902988.3**

(22) Date of filing: **08.10.82**

(86) International application number:
**PCT/JP82/00405**

(87) International publication number:
**WO 83/01195 14.04.83 Gazette 83/09**

(54) **URINE COLLECTOR.**

(30) Priority: 08.10.81 JP 148806/81 u
19.12.81 JP 190176/81 u
27.05.82 JP 76981/82 u

(43) Date of publication of application:
**09.11.83 Bulletin 83/45**

(45) Publication of the grant of the patent:
**04.01.89 Bulletin 89/01**

(84) Designated Contracting States:
**DE FR GB**

(56) References cited:
**GB-A- 667 012
GB-A-2 016 929
GB-A-2 029 764
GB-A-2 050 838
JP-A-50 130 295
JP-A-52 020 691
JP-U-52 163 991
JP-U-55 021 736
US-A-4 179 367
US-A-4 198 979**

(73) Proprietor: TERAUCHI, Ryugo
29-18, Minamiogikubo 4-chome
Suginami-ku Tokyo 167 (JP)
(73) Proprietor: FUKUSHIMA, Kiyoshi
3-4, Kotesashi-cho Tokorozawa-shi
Saitama-ken 359 (JP)

(72) Inventor: TERAUCHI, Ryugo
29-18, Minamiogikubo 4-chome
Suginami-ku Tokyo 167 (JP)
Inventor: FUKUSHIMA, Kiyoshi
3-4, Kotesashi-cho Tokorozawa-shi
Saitama-ken 359 (JP)

(74) Representative: Smith, Norman Ian et al
F.J. CLEVELAND & COMPANY 40-43 Chancery
Lane
London WC2A 1JQ (GB)

Courier Press, Leamington Spa, England.

## Description

This invention relates generally to a urinary appliance for use by the incontinent such as the aged, patients with spinal injuries and the like.

In general, urinary appliances have a urine receiving unit, which is applied to the patient's genitals, so as to receive the urine. A urinal or a urine receiving bag is connected to the urine receiving unit and stores the urine flowing from the urine receiving unit therein.

The conventional urine receiving unit is made of plastic or latex rubber and has the following problem. Since the inside of the urine receiving unit is cut off from the atmosphere and is kept sealed, moisture caused by the urine stays therein and the skin of the genitals can become irritated and sore because of this moisture. Moreover, the urine does not flow smoothly into the urinal or urine bag because the urine inside the urine receiving unit is not at atmospheric pressure. Furthermore, the plastic urine receiving unit is hard and inconvenient for the patient when walking.

GB—A—2016929 discloses a urinary appliance according to the preamble of claim 1.

It is an object of the present invention to provide a urinary appliance which prevents the moisture from remaining inside the urine bag and thereby reduces skin irritation.

According to the present invention there is provided a urinary appliance comprising a urine receiving bag, said urine receiving bag being made of a soft and flexible fabric that is permeable to air and water repellent, and having an opening to be fitted over the patient's genitals at one of its ends characterized by a urine outlet at the other end of said bag, the genital opening of the urine receiving bag being partially closed by an annular sealing sheet, composed of a generally soft, flexible, cloth fabric material, which is permeable to air and water repellent, the sealing sheet having an insertion hole to allow the insertion of the male penis into said urine receiving bag, and to seal the appliance to the body of the user, said sealing sheet being of sufficient width to surround the hole to facilitate sealing of said appliance to the body of the wearer, and said insertion hole being sized and dimensioned to snugly engage the penis.

In an embodiment of a urinary appliance in accordance with the present invention, a urine storage bag incorporating therein a highly water-absorbent resin is connected to the urine receiving bag. The urine is absorbed by this water-absorbent resin, is changed into a gel and thus loses its fluidity. Hence, the patient or wearer of the bag can easily practice so-called "walking rehabilitation". The urine does not flow back from the urine storage bag to the urine receiving bag.

The invention will be described now by way of example only with particular reference to the accompanying drawings. In the drawings;

Figure 1 is a perspective view illustrating a urinary appliance for a male incontinent patient when lying down;

Figure 2 is a front view of the device shown in Figure 1;

Figures 3 through 5 are partly cut-away front views, each illustrating further urinary appliances, being designed so as to enable the patient to walk easily;

Figure 6 is a front view illustrating a first embodiment of the invention in which a modified urine receiving bag is fitted to the device;

Figure 7 is an enlarged perspective view of the urine receiving bag shown in Figure 6; and

Figure 8 is a partly cut-away perspective view illustrating another modified urine receiving bag according to the invention.

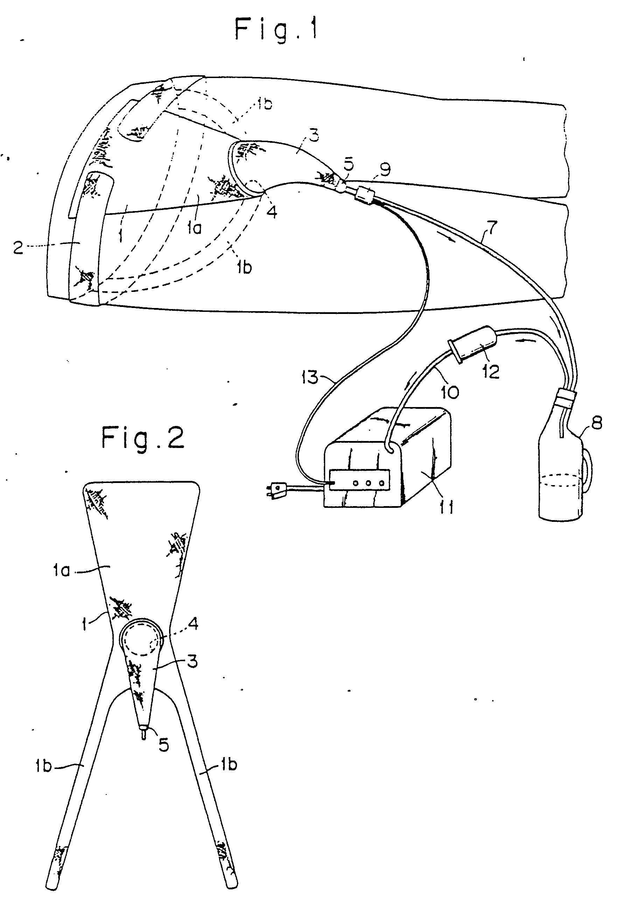

In Figures 1 and 2, a device 1 is shown fitted around the waist of a patient by a belt 2. The device 1 has a front section 1a which fits over the abdominal region of the patient, and two narrow elongated rear sections 1b, 1b which are positioned over the buttocks of the patient. These front and rear sections 1a, 1b, 1b are detachably fitted to the belt 2 by suitable means such as "magic tape" or Velcro. A urine receiving bag 3 is fitted to the front section 1a of the device 1. The urine receiving bag 3 in this embodiment has a shape suitable for the accommodation of the genitals of a man. The urine receiving bag 3 has an opening 4 at one of its ends and a urine outlet 5 at the other. The periphery of this opening 4 is fitted to the device 1. The urine receiving bag 3 is made of a soft and flexible fabric which is permeable to air, and water repellent. The fabric is a woven or non-woven fabric that has been treated with a water-repelling agent. If a woven fabric is used, it is possible to first treat the yarn prior to weaving with the water-repelling agent and then weave the yarn to obtain the woven fabric. The fabric forming the urine receiving bag may be made of a porous resin membrane having through pores. The device 1 may also be formed of the same fabric as that of the urine receiving bag 3.

A urinal 8 is connected to the urine outlet 5 of the urine receiving bag 3 via a tube 7. A sensor is fitted to the urine outlet 5, and a suction unit 11 is connected to the urine outlet 5 via another tube 10 with a deodorizing filter 12 interposed in the tube 10. The sensor 9 is electrically connected to the suction unit 11 via wires 13.

The operation of the urinary appliance having the construction described above will now be described. When the patient urinates when lying down, the urine is received by the urine receiving bag 3 and some of the urine reaches the sensor 9 through the urine outlet 5. The sensor 9 senses the urine and sends a detection signal to the suction unit 11. The suction unit 11 is actuated by this signal so that the air inside the urinal 8 is exhausted through an exhaust port (not shown) formed in the suction unit 11 through the tube 10. Since the inside of the urinal 8 is kept at a negative pressure in this case, the urine inside the urine receiving bag 3 is forced to flow into the urinal 8.

Since the urine receiving bag 3 is permeable to air, its interior is always at atmospheric pressure so that the urine inside the urine receiving bag 3 flows smoothly into the urinal 8 when the suction unit 11 is actuated as described above. Also, because the urine receiving bag 3 is permeable to air, the moisture generated by the urine does not stay inside the urine receiving bag 3 but evaporates outside through the surface of urine receiving bag 3. Thus, the skin of the genitals of the patient can be prevented from becoming sore.

Since the urine receiving bag 3 is also water repellent, the urine neither leaks nor soaks in. Since the urine receiving bag is soft and flexible, it does not feel offensive to the patient. The urine receiving unit 3 for the male patient may be detachably fitted to the device 1.

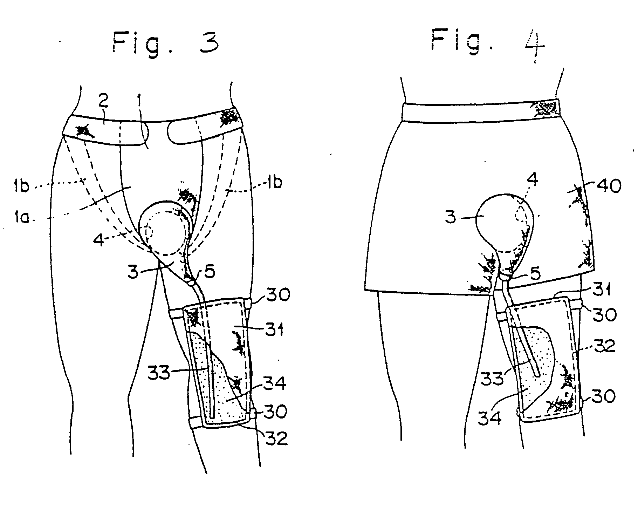

The embodiments of Figures 3 and 5 are specifically designed so that the incontinent patient can walk more easily.

In Figure 3, an external bag 31 is shown fitted to one of the legs of the patient by a band 30. This external bag 31 is made of cloth, plastic or the like. A cloth bag is preferred in this embodiment because the bag comes into direct contact with the skin. The external bag 31 is open at its upper part so that it receives a urine storage bag 32. The urine storage bag 32 is made of rubber or plastic. A tube 33 connected to the urine outlet 5 of the urine receiving bag 3 is inserted into the urine storage bag 32 an is sealed in place. A highly water absorbent resin 34 is placed inside the urine storage bag 32. The following can be listed as examples of the highly water absorbent resin;

(1) starch-acrylonitrile grafted polymer types of highly water-absorbent resins;

(2) cellulose-acrylonitrile grafted polymer types of highly water-absorbent resins;

(3) carboxymethylated polysaccharide-acrylonitrile grafted polymer types of highly water-absorbent resins;

(4) polyacrylic acid types of highly water--absorbent resins;

(5) polyacrylonitrile of highly water-absorbent resins;

(6) non-ionic polymer types of highly water-absorbent resins.

The highly water-absorbent resins may assume various forms such as fibers, cloth, cotton, powder, and so on. The water-absorbent resins 34 may be wrapped in craft paper or the like and is then placed in the urine storage bag 32.

The operation of the urinary appliance shown in Figure 3 will now be described. When the patient urinates when standing, the urine is received by the urine receiving bag 3 and flows into the urine storage bag 32 via the tube 33 due to gravitational force. Since the urine receiving bag 3 is permeable to air, the urine inside the urine receiving bag 3 is at atmospheric pressure and flows smoothly into the urine storage bag 32. The urine is then absorbed by the highly water absorbent resin 34 inside the urine storage bag 32, is changed into a gel and thus loses its fluidity. The patient can walk easily because the urine receiving bag 3 is soft and flexible and because the urine is in the form of a gel inside the urine storage bag 32 and no longer flows.

Even if there are small holes in the urine storage bag 32, the gelled urine will neither leak nor flow back to the urine receiving bag 31. The highly water-absorbent resin can also absorb ammonia released by the decomposition of urine and can thus prevent the occurrence of offensive odors.

The gelled urine is removed from the urine storage bag 32 and is then discarded. The urine storage bag 32 itself may be disposed.

In Figure 4, the urine receiving bag 3 is shown fitted to underpants 40 similar to ordinary underpants.

In Figure 5, the urine receiving bag 3 is shown fitted to elongated underpants 50 and two external bags 31, 31 are also fitted to the underpants 50. Each external bag 31 has placed therein a urine storage bag 32 which in turn has placed therein the highly water-absorbant resin 34. One of the urine storage bags 32 is a spare.

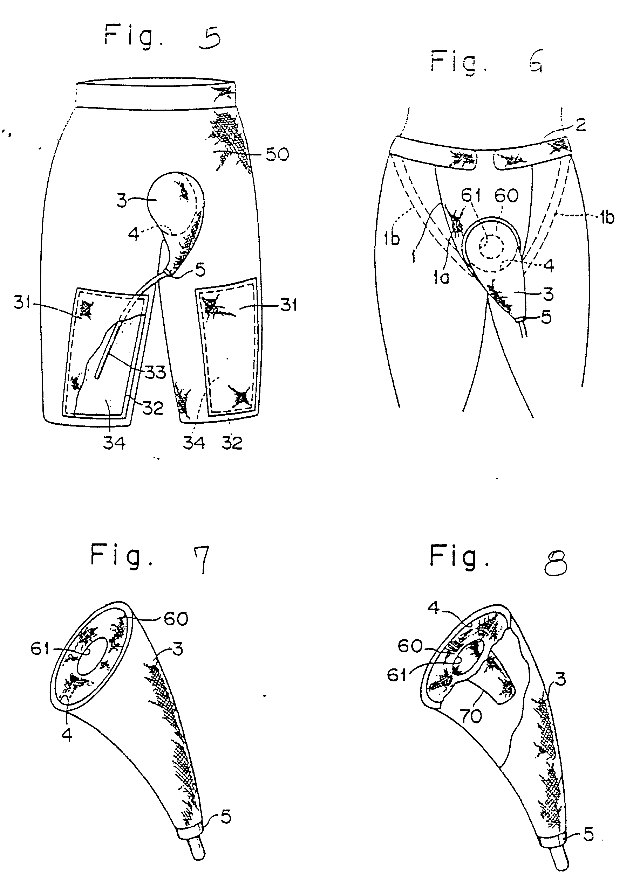

Figures 6 and 7 show a first embodiment of the present invention. In this embodiment, a sealing sheet 60 is fitted around the periphery of the opening 4 of the urine receiving bag 3. The sealing sheet 60 is made of a soft and flexible fabric that is permeable to air, and water repellent. An insertion hole 61 is formed at the center of the sealing sheet 60 and the penis of the male patient is fitted into this insertion hole 61. When the patient urinates in any position such as lying, standing or sitting, using the urinary appliance of this embodiment, the urine passed into the urine receiving bag 3 does not flow back toward the abdominal region of the patient because it is cut off by the sealing sheet 60.

Figure 8 shows a further modified urine receiving bag 30. A cylinder 70 is further fitted around the periphery portion of the insertion hole 61. The cylinder 71 supports the penis of the male patient. The cylinder 70 is made of a soft and flexible fabric that is permeable to air, and water repellent, and is progressively tapered towards its tip. Since the size of the penis varies from patient to patient, the cylinder 70 is cut at a position suitable for that particular patient. Hence, the cylinder 70 can be smoothly and firmly fitted to the penis and can support the same.

**Claims**

1. A urinary appliance comprising a urine receiving bag (3), said urine receiving bag (3) being made of a soft and flexible fabric that is permeable to air and water repellent, and having an opening (4) to be fitted over the patient's genitals at one of its ends characterized by a urine outlet (5) at the other end of said bag, the genital opening (4) of the urine receiving bag (3) being partially closed by an annular sealing sheet (60), composed of a generally soft, flexible, cloth fabric material, which is permeable to air and water repellent, the sealing sheet (60) having an insertion hole (61) to allow the insertion of the male penis into said urine receiving bag (3), and to seal the appliance to the body of the user, said sealing sheet being of sufficient width to surround the hole to facilitate sealing of said appliance to the body of the wearer, and said insertion hole (61) being sized and dimensioned to snugly engage the penis.

2. A urinary appliance according to claim 1 characterized by further comprising a cylinder (70) composed of a soft, flexible, cloth fabric material, said material being air pemerable and water repellent; and wherein said cylinder (70) is fitted around the periphery of the insertion hole (61), and is adapted to extend over a male penis and to snugly engage the penis for sealing purposes.

3. A urinary appliance according to claim 2 characterized in that said cylinder (70) tapers progressively towards its tip with a decreasing diameter.

4. A urinary appliance according to claim 1 characterized in that a urine storage bag (32) is connected to said urine outlet (5) of said urine receiving bag (3).

5. A urinary appliance according to claim 4 characterized in that a highly water-absorbent resin (34) which absorbs and gels the urine is placed inside said urine storage bag (32).

6. A urinary appliance according to claim 1 characterized in that a urinal (8) is connected to said urine outlet (5) of said urine storage bag (3) via a tube (7), a suction unit (11) is connected to said urinal (8) via another tube (10) and a sensor (9) is further fitted to said urine outlet (5) so that when said sensor (9) senses the urine, it actuates said suction unit (11) to extract the air inside said urinal (8) and thereby force the urine inside said urine receiving bag (3) to flow into said urinal (8).

7. A urinary appliance according to claim 2 characterized in that the cylinder (70) extends over a substantial portion of the axial length of the male penis to enhance the seal.

8. A urinary appliance according to claim 1 characterized in that the urine receiving bag (30) is adapted to be removably attached to conventional cloth undergarments (40, 50).

9. A urinary appliance according to claim 8, characterized in that at least one urine storage bag (31) is connected to said urine outlet (5) of said urine receiving bag (3).

10. A urinary appliance according to claim 1, wherein said urine receiving bag is substantially funnel-shaped.

11. A urinary appliance according to claim 1, wherein said urine receiving bag is substantially cup-shaped.

**Patentansprüche**

1. Harnsammler mit einem Urinauffangbeutel (3), wobei der Urinauffangbeutel (3) aus einem weichen und flexiblen Stoff hergestellt ist, welcher luftdurchlässig und wasserabweisend ist und an seinem einen Ende eine Öffnung (4) hat, die passend über die Genitalien des Patienten zu legen ist, gekennzeichnet, durch einen Urinauslaß am anderen Ende dieses Beutels, wobei die Genitalienöffnung (4) des Urinaufnahmebeutels (3) teilweise durch ein ringförmiges Dichtungsblatt (3) geschlossen ist, welches aus einem im wesentlichen weichen, flexiblen Stoffmaterial zusammengesetzt ist, welches luftdurchlässig und wasserabweisend ist, wobei das Dichtungsblatt (60) eine Einführungsöffnung (61) zum Ermöglichen des Einführens des männlichen Penis in den Urinauffangbeutel (3) besitzt, und um den Harnsammler gegenüber dem Körper des Benutzers abzudichten, wobei das Dichtungsblatt eine ausreichende Breite hat, um die Öffnung zu umgeben und das Abdichten des Harnsammlers gegen den Körper des Trägers zu erleichtern, und wobei die Einführungsöffnung (61) so geformt und bemessen ist, daß sie den Penis eng erfaßt.

2. Harnsammler nach Anspruch 1, dadurch gekennzeichnet, daß er ferner einen Zylinder (70) umfaßt, der aus einem weichen, flexiblen Stoffmaterial zusammengesetzt ist, wobei dieses Material luftdurchlässig und wasserabweisend ist, und daß der Zylinder (70) rund um den Umfang der Einführungsöffnung (61) befestigt und geeignet ist, sich über einen männlichen Penis zu erstrecken und den Penis zu Dichtzwecken eng anliegend zu erfassen.

3. Harnsammler nach Anspruch 2, dadurch gekennzeichnet, daß sich der Zylinder (70) in Richtung auf eine Spitze mit abnehmendem Durchmesser zunehmend verjüngt.

4. Harnsammler nach Anspruch 1, dadurch gekennzeichnet, daß mit dem Urinauslaß (5) des Urinauffangbeutels (3) ein Urinsammelbeutel (32) verbunden ist.

5. Harnsammler nach Anspruch 4, dadurch gekennzeichnet, daß in dem Urinsammelbeutel (32) ein stark wasserabsorbierendes Kunstharz (34) angeordnet ist, welches den Urin absorbiert und gelieren läßt.

6. Harnsammler nach Anspruch 1, dadurch gekennzeichnet, daß mit dem Urinauslaß (5) des Urinauffangbeutels (3) über einen Schlauch (7) eine Urinflasche (8) verbunden ist, daß mit der Urinflasche (8) über einen weiteren Schlauch (10) eine Saugeinheit (11) verbunden ist und daß an dem Urinauslaß (5) ferner ein Sensor (9) angebracht ist, derart, daß der Sensor, wenn er das Vorhandensein des Urins erfaßt, die Saugeinheit (11) betätigt, um Luft aus der Urinflasche (8) abzusaugen und damit den Urin im Inneren des Urinauffangbeutels (3) zu zwingen, in die Urinflasche (8) zu fließen.

7. Harnsammler nach Anspruch 2, dadurch gekennzeichnet, daß sich der Zylinder (70) über einen beträchtlichen Teil der axialen Länge des männlichen Penis erstreckt, um die Dicktwirkung zu verbessern.

8. Harnsammler nach Anspruch 1, dadurch gekennzeichnet, daß der Urinauffangbeutel (30) geeignet ist, lösbar an konventioneller Stoff-Unterwäsche (40, 50) befestigt zu werden.

9. Harnsammler nach Anspruch 8, dadurch gekennzeichnet, daß mit dem Urinauslaß (5) des Urinauffangbeutels (3) mindestens ein Urinsammelbeutel (32) verbunden ist.

10. Harnsammler nach Anspruch 1, dadurch gekennzeichnet, daß der Urinauffangbeutel im wesentlichen trichterförmig ausgebildet ist.

11. Harnsammler nach Anspruch 1, dadurch gekennzeichnet, daß der Urinauffangbeutel im wesentlichen becherförmig ausgebildet ist.

**Revendications**

1. Dispositif urinaire comprenant un sac (3) de réception de l'urine, ledit sac (3) de réception de l'urine étant en tissu souple et flexible et perméable à l'air et imperméable à l'eau, et ayant une ouverture (4) adaptable aux parties génitales du patient à une des ses extrémités, caractérisé en ce qu'il comprend une sortie (5) d'urine à l'autre extrémité dudit sac, l'ouverture (4) vers les parties génitales du sac (3) de réception de l'urine étant fermée par une feuille (60) annulaire d'étanchéité, composée d'un matériau en tissu de vêtement généralement doux, flexible, qui est perméable à l'air et imperméable à l'eau, la feuille (60) d'étanchéité ayant un trou (61) d'insertion pour permettre l'insertion du pénis mâle dans ledit sac (3) de réception d'urine, et fermer le dispositif de façon étanche avec le corps de l'utilisateur, ladite feuille d'étanchéité étant de largeur suffisante pour entourer le trou afin de faciliter la fermeture étanche dudit dispositif au corps du porteur, et ledit trou d'insertion (61) étant calculé et dimensionné pour coopérer confortablement et de façon ajustée avec le pénis.

2. Dispositif urinaire selon la revendication 1, caractérisé en ce qu'il comporte de plus un cylindre (70) composé en matériau de tissu de vêtement doux flexible, ledit matériau étant perméable à l'air et imperméable à l'eau; et dans lequel ledit cylindre (70) est agencé pour coopérer autour de la périphérie du trou d'insertion (61), et est adapté pour s'étendre sur un pénis mâle et pour coopérer de façon confortable, et ajusté avec le penis en vue d'assurer une bonne étanchéité.

3. Dispositif urinaire selon la revendication 2, caractérisé en ce que le cylindre (70) a une forme conique de diamètre progressivement décroissant vers son extrémité.

4. Dispositif urinaire selon la revendication 1, caractérisé en ce que un sac (32) de stockage de l'urine est connecté par la sortie d'urine (5) dudit sac (3) de réception de l'urine.

5. Dispositif urinaire selon la revendication 4, caractérisé en ce que une résine (34) hautement absorbante d'eau qui absorbe et gélifie l'urine est placée à l'intérieur audit sac de stockage de l'urine (32).

6. Dispositif urinaire selon la revendication 1, caractérisé en ce que un urinal est raccordé à ladite sortie d'urine (5) dudit sac (3) de stockage d'urine via un tube (7), une unité (11) d'aspiration est connectée audit urinal (8) via un autre tube (10) et un capteur (9) est de plus adapté à ladite sortie (5) d'urine de sorte que quand ledit capteur (9) détecte l'urine, il actionne ladite unité de d'aspiration (11) pour extraire l'air à l'intérieur dudit urinal (8) et de cette façon force l'urine à l'intérieur dudit sac (3) de réception de l'urine à s'écouler dans ledit urinal (8).

7. Dispositif urinaire selon la revendication 2, caractérisé en ce que le cylindre (70) s'étend sur une portion substantielle de la longueur axiale du pénis mâle pour améliorer l'étanchéité.

8. Dispositif urinaire selon la revendication 1, caractérisé en ce que le sac (30) de réception d'urine est agencé pour être attaché de façon amovible à des sous-vêtements (40, 50) de type connu.

9. Dispositif urinaire selon la revendication 8, caractérisé en ce que au moins un sac (31) de stockage de l'urine est raccordé à ladite sortie (5) d'urine dudit sac (3) de réception d'urine.

10. Dispositif urinaire selon la revendication 1, caractérisé en ce que ledit sac de réception d'urine est de forme sensiblement en entonnoir.

11. Dispositif urinaire selon la revendication 1, caractérisé en ce que ledit sac de réception de l'urine est de forme sensiblement en cuvette.

EP 0 093 175 B1

# Fig.1

# Fig.2

1

# Fig. 3

# Fig. 4

Fig. 5

Fig. 6

Fig. 7

Fig. 8